# EUROPÄISCHE PATENTANMELDUNG

(11) **EP 3 735 997 A1**
(43) Veröffentlichungstag der Anmeldung: **11.11.2020**
(21) Anmeldenummer: 19173203.1
(22) Anmeldetag: 08.05.2019
(51) Int. Cl.: A61M 5/14, A61M 5/20, A61M 5/31

(54) **ELEKTRONISCHES ZUSATZMODUL FÜR INJEKTIONSGERÄTE**

(71) Anmelder: Ypsomed AG, 3401 Burgdorf (CH)
(72) Erfinder: Allenspach, Marcel, 3400 Burgdorf (CH); Hirschel, Jürg, 3007 Bern (CH); Bernhard, Mario, 3400 Burgdorf (CH); Schrul, Christian, 3400 Burgdorf (CH)
(74) Vertreter: Kleiner, Stefan

(57) **Zusammenfassung**

Die Erfindung betrifft ein elektronisches Zusatzmodul mit einer Vorrichtung zur lösbaren Befestigung des Zusatzmoduls auf einem Injektionsgerät, welche zur axialen Fixierung von Zusatzmodul und Injektionsgerät nicht auf eine Struktur in einer Oberfläche des Injektionsgerätes angewiesen ist. Ein Gerätegehäuse des Injektionsgeräts weist auf einer ersten Aussenseite eine zur Längsachse parallele Auflagefläche und eine der ersten Seite bezüglich der Längsachse gegenüberliegende zweite Aussenseite auf. Das Zusatzmodul umfasst ein in einem Modulgehäuse des Zusatzmoduls radial verschiebbar gelagertes Modulelement mit einer Anpressfläche, deren Form an die Form der Auflagefläche angepasst ist und diese im aufgesetzten Zustand kontaktiert. Das Zusatzmodul umfasst einen an der zweiten Seite des Gerätegehäuse abstützenden Federmechanismus zum haftreibungserzeugenden, zumindest abschnittweise vollflächigen Anpressen der Anpressfläche auf die Auflagefläche.

## Beschreibung

### TECHNISCHES GEBIET

Die vorliegende Erfindung betrifft das Gebiet der medizinischen Injektionsgeräte zur Verabreichung von flüssigen Substanzen, insbesondere von Medikamenten oder medizinischen Substanzen wie Insulin- und Hormonpräparationen. Die Erfindung bezieht sich auf ein portables elektronisches Zusatzmodul zum Aufsetzen auf ein medizinisches Injektionsgerät.

### HINTERGRUND

Unterschiedliche Infusions- und Injektionsgeräte können Patienten bei einer kontrollierten subkutanen oder intramuskulären Verabreichung einer Dosis eines Medikamentes aus einem Reservoir unterstützen. Während bei Infusionssystemen eine Kanüle während längerer Zeit im Körper des Patienten verbleibt werden Injektionsgeräte nach jeder Abgabe wieder von der Injektionsstelle entfernt. In Einweg-Injektionsgeräten ist das Reservoir, beispielsweise eine Fertigspritze, nicht dazu vorgesehen, durch den Patienten ersetzt oder wiederaufgefüllt zu werden. In Mehrweg-Injektionsgeräten dagegen ist kann das Reservoir, beispielsweise eine Karpule, durch den Patienten wieder befüllt oder ersetzt werden. Ein automatisches Injektionsgerät verfügt über einen Motor oder eine gespannte Feder als Energiequelle zum Antrieb einer Kolbenstange und zur Bewegung eines Kolbens im Reservoir, während bei einer Ausschüttung mit einem manuellen Injektionsgerät die Kolbenstange unmittelbar durch eine Kraft des Patienten bewegt wird.

Diabetes wird oft durch Abgabe von Insulin mittels eines Injektionsgerätes in Form eines Insulin-Pens mit einstellbarer Dosis behandelt. Der Pen verfügt über ein längliches Gehäuse mit einem distalen Ende zur Aufnahme einer Kanüle oder Hohlnadel und mit einem der Injektionsstelle abgewandten proximalen Ende. Ein Insulin-Pen kann als Ein- oder Mehrweg-Pen, automatisch oder manuell betrieben werden. Die abzugebende Insulindosis wird normalerweise eingestellt durch Drehen eines Dosierknopfs und gleichzeitiger Kontrolle einer Dosisanzeige des Insulin-Pens. Um eine Insulinabgabe in Echtzeit zu überwachen, beispielsweise zur Verhinderung von Fehlmanipulationen, oder zur Nachverfolgung von mehreren Abgaben über einen längeren Zeitraum, können Informationen betreffend Insulin-Typ, Dosisgrösse, und Zeitpunkt sowie weiterer Umstände jeder Dosisabgabe gesammelt werden.

EP18173675.2 zeigt zu diesem Zweck ein elektronisches Zusatzmodul, welches seitlich auf ein Injektionsgerät aufgesetzt und daran befestigt oder geklemmt werden kann mittels einer Schnappverbindung. Ein Schnappmechanismus umfasst zwei elastische Flügel am Zusatzmodul welche einen Aufnahmebereich für das Injektionsgerät definieren. Das Zusatzmodul umfasst einen Sensor zur Detektion eines Zustandes des Injektionsgerätes und/oder zur Überwachung eines laufenden Ausschüttvorganges.

WO 2007/107564 A1 beschreibt ein Zusatzmodul für ein Injektionsgerät mit einem am proximalen Ende des Injektionsgerätes angeordneten Dosiseinstellknopf. Vor oder nach dem Gebrauch des Injektionsgerätes wird ein distales Ende des Injektionsgeräts durch eine Gerätekappe geschützt, welche in ein Kopplungselement des Injektionsgerätes greift und dadurch gegen unbeabsichtigtes Lösen in distale Richtung gefeit ist. Das Zusatzmodul kann nach Entfernen der Gerätekappe ebenfalls in das Kopplungselement eingreifen und dadurch das Modul gegen eine proximale Bewegung sichern. Das Zusatzmodul wiederum weist eigene Kopplungselemente auf, welche es erlauben, eine angepasste Gerätekappe bei montiertem Zusatzmodul an diesen zu befestigen.

Der Begriff "Medikament" oder "medizinische Substanz" umfasst in diesem Zusammenhang jede fliessfähige medizinische Formulierung, welche geeignet ist zur kontrollierten Verabreichung mittels einer Kanüle oder Hohlnadel, beispielsweise eine Flüssigkeit, eine Lösung, ein Gel oder eine feine Suspension enthaltend einen oder mehrere medizinische Wirkstoffe. Ein Medikament kann also eine Zusammensetzung mit einem einzigen Wirkstoff oder eine vorgemischte oder co-formulierte Zusammensetzung mit mehreren Wirkstoffen aus einem einzelnen Behälter sein. Der Begriff umfasst insbesondere Arzneien wie Peptide (z.B. Insuline, Insulin enthaltende Medikamente, GLP-1 enthaltende sowie abgeleitete oder analoge Zubereitungen), Proteine und Hormone, biologisch gewonnene oder aktive Wirkstoffe, Wirkstoffe auf Basis von Hormonen oder Genen, Nährformulierungen, Enzyme und weitere Substanzen sowohl in fester (suspendierter) oder flüssiger Form. Der Begriff umfasst weiter auch Polysaccharide, Vakzine, DNS oder RNS oder Oligonukleotide, Antikörper oder Teile von Antikörpern sowie geeignete Basis-, Hilfs- und Trägerstoffe.

### DARSTELLUNG DER ERFINDUNG

Es ist eine Aufgabe der Erfindung ein elektronisches Zusatzmodul so auf einem Injektionsgerät lösbar zu befestigen dass Bewegungen des Injektionsgeräts unverfälscht auf eine Sensoreinheit im Zusatzmodul übertragen werden. Diese Aufgabe wird gelöst durch ein Zusatzmodul mit den Merkmalen des unabhängigen Anspruchs. Bevorzugte Ausführungsformen der Erfindung sind Gegenstand der abhängigen Ansprüche.

Erfindungsgemäss wird ein elektronisches Zusatzmodul auf ein Injektionsgerät in Richtung einer Längsachse, welche ein einstechseitiges distales Ende mit einem gegenüberliegenden proximalen Ende des Injektionsgeräts verbindet, lösbar aufgesetzt oder aufgeschoben, beziehungsweise das Injektionsgerät in einen Aufnahmebereich des Zusatzmoduls eingeführt oder eingeschoben. Das Injektionsgerät umfasst ein Gerätegehäuse welches eine Abgabemechanik zur parenteralen subkutanen oder intramuskulären Abgabe von Medikamenten aus einem Behälter des Injektionsgerätes umgibt. Das Gerätegehäuse weist auf einer ersten Aussenseite als Auflageseite des Gerätegehäuses eine zur Längsachse parallele Auflagefläche auf, umfassend einen Oberflächenabschnitt des Gerätegehäuses, bevorzugt ein Segment einer Kreiszylinderoberfläche. Das Gerätegehäuse weist weiter eine der ersten Seite bezüglich der Längsachse gegenüberliegende zweite Aussenseite auf. Das Zusatzmodul umfasst ein in einem Modulgehäuse des Zusatzmoduls radial, das heisst senkrecht oder quer zur Längsachse verschiebbar gelagertes Modulelement. Das Modulelement hat eine Anpressfläche, deren Form an die Form der Auflagefläche angepasst ist und diese im aufgesetzten Zustand von Zusatzmodul und Injektionsgerät kontaktiert. Das Zusatzmodul umfasst einen an der zweiten Seite des Gerätegehäuse abstützenden oder eingreifenden Federmechanismus zum haftreibungserzeugenden, zumindest abschnittweise vollflächigen Anpressen der Anpressfläche auf die Auflagefläche und zum Festklemmen des Zusatzmoduls auf dem Injektionsgerät.

Durch das flächige Anpressen wird sichergestellt, dass Bewegungen des Injektionsgerätes unverfälscht, das heisst möglichst unverzerrt und minimal gedämpft und verzögert auf das Modulelement übertragen werden, insbesondere auch Bewegungskomponenten in radialer Richtung. Die Bewegungen des Injektionsgerätes umfassen dabei sowohl Bewegungen des Geräts im Raum, welche durch einen Anwender ausgeführt werden, als auch Bewegungen des Geräts, welche durch einen Mechanismus im Injektionsgerät verursacht werden. Dazu zählen beispielsweise Schwingungen, Vibrationen oder Schallwellen im Injektionsgerät, welche über die Auflagefläche des Gerätegehäuses und die angepresste Anpressfläche auf das Modulelement übertragen werden. Idealerweise ist die Anpressvorrichtung auch zur alleinigen axialen Fixierung von Zusatzmodul und Injektionsgerät geeignet, so dass zu diesem Zweck keine Nocken oder Schnapper am Zusatzmodul notwendig sind.

Das Modulelement ist als Ganzes geführt und radial bewegbar, und nicht etwa gelenkig mit dem Modulgehäuse verbunden oder einstückig mit dem Modulgehäuse ausgeführt wie dies bei elastischen Modulflügeln zum seitlichen Aufschnappen des Zusatzmoduls auf das Injektionsgerät der Fall ist. Eine allfällig im Bereich der Auflagefläche vorhandene Struktur wie beispielsweise ein Flansch, ein Nocken, eine Ausnehmung, oder eine Öffnung in der Oberfläche des Injektionsgerätes kann zur axialen Befestigen des Zusatzmoduls unbeachtet bleiben. Die Struktur steht daher auch bei aufgesetztem Zusatzmodul für ihren ursprünglichen Zweck, beispielsweise zur Fixierung einer Gerätekappe, zur Verfügung.

Eine Ausdehnung der Anpressfläche in Längsrichtung entspricht mindestens der halben und bevorzugt annähernd der gesamten Länge des Zusatzmoduls. Auflagefläche und/oder Anpressfläche können in Längsrichtung segmentiert sein und mehrere kontaktbildende Abschnitte umfassen, wobei allfällige Kanten oder Stufen zwischen Abschnitten der Auflagefläche nicht zur Fixierung des Zusatzmoduls genutzt werden. Auflagefläche und Anpressfläche weisen eine in Längsrichtung unveränderte Kontur oder Querschnitt senkrecht zur Längsachse auf, und bilden somit ein Segment eines Mantels eines geometrischen Zylinders. Auflagefläche und Anpressfläche können dabei Kanten in Längsrichtung zwischen daran seitlich anschliessenden Flächenabschnitten aufweisen, die Auflagefläche kann ein Segment einer um die Längsachse rotationssymmetrischen Oberfläche des Injektionsgeräts sein.

In einer bevorzugten Ausführungsform der Erfindung umfasst das Zusatzmodul eine Sensoreinheit welche auf Bewegungen des Injektionsgeräts anspricht und diese zuverlässig detektiert. Die Sensoreinheit umfasst dazu mindestens einen ein- oder mehrachsigen Beschleunigungssensor, einen ein- oder mehrachsigen Gyroskop-sensor, einen Trägheitssensor, ein piezobasiertes Körperschallmikrofon, oder einen vergleichbaren Sensor zur Detektion von Bewegungen des Injektionsgerätes. Die Sensoreinheit ist unbeweglich fest und dauerhaft auf der Moduleinheit befestigt, so dass Bewegungen der Auflagefläche unverfälscht, das heisst möglichst ungedämpft und unverzerrt über die Anpressfläche bis zur Sensoreinheit übertragen werden. Das Zusatzmodul kann weitere, nicht-mechanische Sensoren umfassen, sowie eine Prozessoreinheit zur Auswertung und/oder Verarbeitung eines Signals der Sensoreinheit und daraus zur Erkennung eines Vorganges im Injektionsgerät, beispielsweise eines Ausschüttklicks. Das Zusatzmodul umfasst weiter einen Energiespeicher zur Energieversorgung von Sensor- und/oder Prozessoreinheit, und eine Kommunikationseinheit zur drahtlosen Kommunikation von Signalen der Sensoreinheit und/oder von Daten der Prozessoreinheit.

In einer bevorzugten Variante der Erfindung sind der Federmechanismus und die Lagerung des Modulelements so ausgebildet, dass das Modulelement während einer Aufsetzbewegung des Zusatzmoduls auf das Injektionsgerät um eine quer zur Längsrichtung angeordnete Achse leicht verkippt und die Anpressfläche dadurch nicht parallel zur Auflagefläche ist. Erst am Ende der Aufsetzbewegung kontaktiert die Anpressfläche vollflächig die Auflagefläche, wodurch eine maximale Haftreibungskraft entsteht. Während der Verkippung wird die Anpressfläche bei der Aufsetzbewegung über eine Auslenkkante eines Absatzes am Beginn der Auflagefläche geschoben. Dies erzeugt deutlich geringere Reibungskräfte als ein vollflächiger Gleitreibungskontakt, so dass ein Anwender erst am Ende der Aufsetzbewegung eine maximale Aufsetzkraft aufwenden muss. Ein deutliches, zur Aufsetzbewegung nichtlineares Ansteigen der aufzuwendenden Aufsetzkraft kann der Anwender als Zeichen einer vollständigen Aufsetzung erkennen, ähnlich einem Kraftverlauf mit einem Schnappmechanismus am Ende der Aufsetzbewegung. Ebenso sinkt beim Lösen des Zusatzmoduls vom Injektionsgerät nach initialem Überwinden einer maximalen Haft- oder Gleitreibungskraft die Lösekraft nichtlinear ab. Durch eine bestimmte Ausbildung des Federmechanismus kann der Kraftverlauf während dem Aufsetzen also gestaltet werden.

In einer bevorzugten Weiterbildung umfassen ein erstes, proximales oder distales, Ende der Anpressfläche des Modulelements und eine Auslenkkante am zweiten, distalen oder proximalen, Ende der Auflagefläche des Gerätegehäuses mindestens eine gegenüber einer Senkrechten zur Längsachse abgeschrägte Führungsfläche. Dadurch wird zu Beginn der Aufsetzbewegung, beim Aufeinandertreffen der beiden Enden, das erste Ende der Anpressfläche radial nach aussen gelenkt und anschliessend im verkippten Zustand über die Auslenkkante geschoben. Bei einer bezüglich der Mitte des Modulelements symmetrischen Anordnung des Federmechanismus nimmt eine Verkippung oder ein Neigungswinkel des Modulelementes zu bis besagte Mitte die Auslenkkante passiert, und anschliessend wieder ab.

In einer vorteilhaften Variante der Erfindung umfasst das Zusatzmodul einen Aufnahmebereich in Form einer an den Querschnitt des Injektionsgerätes angepassten

Öffnung zur Aufnahme des Injektionsgerätes, so dass im montierten Zustand die erste Seite und zumindest ein Teil der gegenüberliegenden zweiten Seite des Injektionsgerätes vom Zusatzmodul umfasst sind. Der Federmechanismus umfasst ein starres Verbindungselement bevorzugt als Teil des Modulgehäuses, welches an der zweiten Seite des Injektionsgerätes abstützt und eine Anpresskraft oder Gegenkraft auf diese überträgt, sowie ein elastisches Federelement, welches kräfteübertragend zwischen dem Verbindungselement und dem Modulelement vorgesehen ist. Das Federelement ist wirkt bevorzugt auf Druck und wird bei Aufsetzen des Zusatzmoduls zwischen dem ausgelenkten Modulelement und dem starren Verbindungselement komprimiert. Alternativ kann das elastische Federelement auch auf Zug wirken und beim Aufsetzen des Zusatzmoduls gesehnt werden. In beiden Fällen reicht das Modulelement in einer Ausgangsposition vor Beginn der Aufsetzbewegung in den lichten Bereich des Aufnahmebereichs, so dass eine radiale Auslenkung durch die Führungsflächen und ein Spannen des Federelements bei Beginn der Aufsetzbewegung erfolgen kann. Im Ausgangszustand ist ein Abstand zwischen der Anpressfläche und einem Ende des Verbindungselements, welches zur Kontaktierung der zweiten Seite des Injektionsgerätes vorgesehen ist, kleiner als ein Abstand zwischen der ersten Seite und der zweiten Seite des Injektionsgerätes.

In einer bevorzugten Weiterbildung ist das elastische Federelement in Richtung der Längsachse derart ausgedehnt oder unterteilt, dass es an mehreren über die Länge verteilten Stellen an Verbindungselement und Modulelement angreifen kann und die Anpresskraft nicht nur an einer Stelle erzeugt wird. Beispielsweise umfasst das Federelement einen Federdraht, oder ein Federblatt, welcher in der Mitte das Verbindungselement und an den beiden Enden das Modulelement kontaktiert. Ebenso gut kann der Federdraht in seiner Mitte auf dem Modulelement aufliegen und mit seinen Enden am Verbindungselement abstützen. Das Federelement kann auch mehrere über die Länge des Modulelements verteilte Spiralfedern umfassen.

Falls das Zusatzmodul auf ein um die Längsachse rotationssymmetrisches Injektionsgerät aufgesetzt werden soll ist in vorteilhafter Weise ein Rotationsbegrenzungselement zur relativen Positionierung von Zusatzmodul und Injektionsgerät in Rotationsrichtung um die Längsachse vorgesehen. Das Element schränkt eine Verdrehung oder Rotation auf einen Bruchteil einer vollen Umdrehung ein, bevorzugt auf einen Winkel von höchstens zwanzig Grad. Das Rotationsbegrenzungselement kann einen Schnapper am Zusatzmodul umfassen, welcher in Öffnungen oder Ausnehmungen an der Aussenseite des Injektionsgerätes eingreifen kann, beispielsweise in ein Fenster des Karpulenhalters oder einer Dosisanzeige. Alternativ können auch ein Nocken und geeignete Führungsschienen an Zusatzmodul und Injektionsgerät zur Rotationsbegrenzung genutzt werden.

In einer bevorzugten Variante weist die Anpressfläche einen konkaven Querschnitt auf in einer Ebene senkrecht zur Längsachse, welcher an einen konvexen oder gewölbten, nichtebenen Auflageabschnitt des Injektionsgeräts angepasst ist. Die beiden Flächen können dabei sogar Kanten oder Falten in Richtung der Längsachse aufweisen. Das Modulelement ist bezüglich einer die Längsachse umfassenden Symmetrieebene zumindest näherungsweise symmetrisch ausgebildet, und das Zusatzmodul umfasst einen ebenen Elektronikträger. Der Federmechanismus des Zusatzmoduls weist zwei Federelemente auf, insbesondere zwei parallele Federdrähte, welche bezüglich der Symmetrieebene symmetrisch angeordnet sind, und zwar zwischen der Anpressfläche und dem Elektronikträger. Dadurch sind die Federelemente platzsparend untergebracht in einem Volumen mit bezüglich dem Elektronikträger nicht-konstanter Ausdehnung, welches für die Elektronikkomponenten nur bedingt nutzbar ist. Der Elektronikträger wird bevorzugt mit minimalem radialem Abstand zur Anpressfläche angeordnet, und sämtliche Elektronikkomponenten auf einer der Anpressfläche abgewandten Seite bestückt. Weiter bevorzugt wird in besagtem Volumen zwischen Anpressfläche und Elektronikträger auch ein Energiespeicher angeordnet, welcher beispielsweise längliche, bevorzugt zylinderförmige Batterien umfasst.

In einer bevorzugten Variante umfasst das Zusatzmodul einen Gerätekappensensor in Form eines mechanischen Schalters zur Detektion einer Entfernung einer Gerätekappe des Injektionsgerätes, und einen kapazitiven Berührungssensor zur Detektion einer Berührung des Zusatzmoduls durch einen Anwender. Das Zusatzmodul wird aktiviert und/oder aus einem Schlaf- oder Energiesparmodus erweckt entweder wenn eine Entfernung der Gerätekappe detektiert wird, oder, bei entfernter Gerätekappe und länger dauernder Inaktivität, wenn eine Berührung des Berührungssensor registriert wird.

In einer bevorzugten Variante weist das Zusatzmodul eine senkrecht zur Längsachse orientierte Anschlagfläche auf, zur Begrenzung einer axialen Relativbewegung von Zusatzmodul und Injektionsgerät in eine Aufsetzrichtung. Durch Anschlag der Anschlagfläche an einer Stoppfläche des Injektionsgeräts werden Auflagefläche und Anpressfläche dadurch axial positioniert.

In einer bevorzugten Weiterbildung ist die axiale Relativbewegung eine Aufsetzbewegung des Zusatzmoduls auf das Injektionsgerät in proximale Richtung, von vorne über den Karpulenhalter, wobei die axiale Anschlagfläche an einer Stoppfläche umfassend einen Flansch am Karpulenhalter des Injektionsgerätes anschlägt. Ein Dosiswahlknopf, und optional auch eine Dosisanzeige, an einem proximalen Ende des Injektionsgerätes wird dabei nicht durch das Zusatzmodul berührt oder umfasst, so dass der Aufnahmebereich nicht auf die Dimension des Dosiswahlknopf abgestimmt sein muss und dieser somit eine grössere Ausdehnung senkrecht zur Längsachse aufweisen kann als das Gehäuse des Injektionsgerätes.

Alternativ dazu und insbesondere geeignet für Injektionsgeräte wie Autoinjektoren ohne Bedienelemente zur Dosiswahl oder zur Auslösung am proximalen Ende kann die axiale Relativbewegung eine Aufsetzbewegung des Zusatzmoduls auf das Injektionsgerät in distale Richtung sein. Dabei weist das Zusatzmodul einen Aufnahmebereich zur Aufnahme des Injektionsgerätes auf in Form eines Sacklochs mit einem Boden senkrecht zur Längsrichtung als axiale Anschlagfläche. Diese kontaktiert im aufgesetzten Zustand eine proximale Endfläche des Injektionsgeräts als Stoppfläche.

Ein Zusatzmodul nach der Erfindung kann in vorteilhafter Weise wiederholt eingesetzt werden zur Überwachung eines Gebrauchs oder Einsatzes von automatischen Einweg-Injektionsgeräten oder Autoinjektoren. Insbesondere ist das Zusatzmodul geeignet für einen Retrofit-Einsatz bei existierenden Injektionsgeräten, welche für eine Anpassung oder Modifikation nicht zur Verfügung stehen. In dieser Konfiguration sind im Injektionsgerät keine Sensoren vorgesehen zur Erfassung oder Bearbeitung von Sensordaten über die Operation des Gerätes, ebenso wenig eine Kommunikationsschnittstelle zur Übermittlung dieser Daten an einen Empfänger. Dementsprechend müssen die Sensorelemente im Zusatzgerät derart ausgestaltet und positioniert werden, dass sie Zustandsänderungen oder Primärsignale von innerhalb des Injektionsgerätes feststellen können.

Für den Fachmann sind direkt und offensichtlich weitere Ausführungsformen und Ausgestaltungen erkennbar, welche sich aus Kombinationen der beschriebenen Beispiele oder Kombinationen der beschriebenen Beispiele mit dem allgemeinen Fachwissen des Fachmanns ergeben.

### FIGUREN

In Zusammenhang mit den angehängten Figuren werden nachfolgend bevorzugte Ausführungsformen der Erfindung beschrieben. Diese sollen grundsätzliche Möglichkeiten der Erfindung aufzeigen und keinesfalls einschränkend ausgelegt werden.
Fig.1 zeigt einen Längsschnitt durch ein Injektionsgerät und ein Zusatzmodul;
Fig.2 illustriert ein Aufsetzen des Zusatzmoduls auf das Injektionsgerät;
Fig.3 zeigt ein Einweg-Injektionsgerät mit variabler Dosisabgabe;
Fig.4 zeigt Einzelteile eines Zusatzmoduls in einer Explosionsdarstellung;
Fig.5 zeigt zwei Querschnitte durch das Zusatzmodul aus Fig.4, und
FIg.6 zeigt zwei Längsschnitte durch das Zusatzmodul aus Fig.4.

### FIGURENBESCHREIBUNG

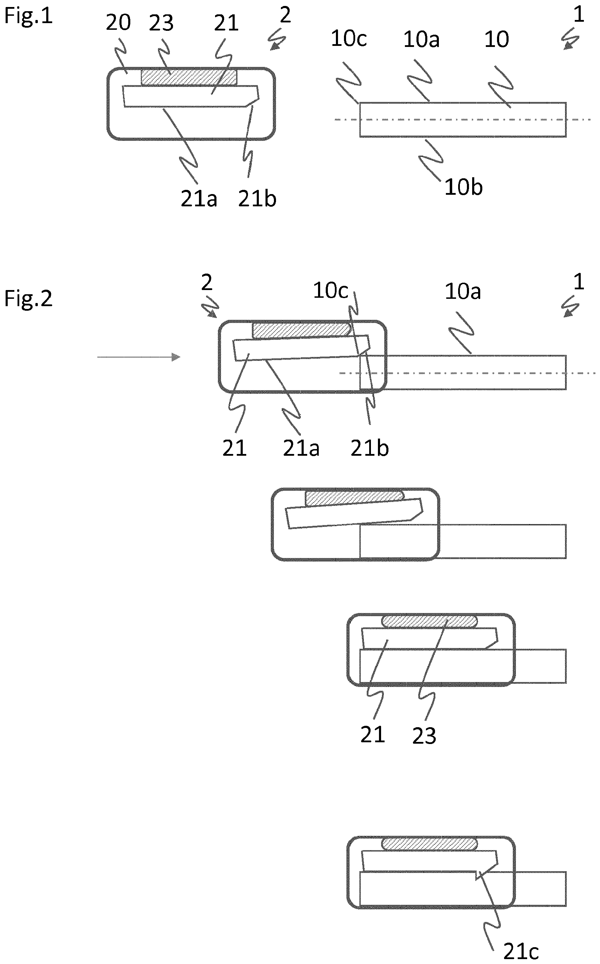

Fig.1 illustriert Elemente der Erfindung anhand einer schematischen Darstellung eines Längsschnitts durch ein Injektionsgerät 1 mit einer als horizontale strichpunktierte Linie dargestellten Längsachse. Das Injektionsgerät umfasst ein Gerätegehäuse 10 mit einer zur Längsachse parallelen Auflagefläche 10a auf einer ersten Seite, oder Oberseite, des Injektionsgeräts und einer der ersten Seite gegenüberliegenden zweiten Seite, oder Unterseite, mit einer Gegenfläche 10b. Neben einem ersten Ende, oder einer ersten Begrenzung in Längsrichtung, der Auflagefläche 10a befindet sich eine Auslenkkante 10c, welche in Fig.1 senkrecht zur Schnittebene ausgerichtet ist. Ein Zusatzmodul 2 umfasst ein in einem Modulgehäuse 20 senkrecht zur Längsachse verschiebbar gelagertes Modulelement 21 mit einer Anpressfläche 21a und mit einer Führungsfläche 21b neben einem zweiten Ende, oder einer zweiten Begrenzung in Längsrichtung, der Anpressfläche 21a. Das Zusatzmodul umfasst ein elastisches Federelement 23 (schraffiert dargestellt) als Teil eines in einem aufgesetzten Zustand des Zusatzmoduls an der Gegenfläche 10b des Gerätegehäuse abstützenden Federmechanismus, welcher die Anpressfläche 21a senkrecht zur Längsachse zur Auflageflache 10a hin presst, drückt oder zieht. Die in Fig.1 als gerade Linien sichtbaren Auflagefläche 10a und Anpressfläche 21a haben eine aneinander angepasste Form mit in einer Ebene senkrecht zur Längsachse identischen Konturen. Durch den daraus resultierenden Flächenkontakt werden auch Bewegungen und insbesondere Vibrationen mit Komponenten senkrecht zur Längsachse optimal vom Gehäuse 10 des Injektionsgeräts auf das Modulelement 21 übertragen.

Fig.2 illustriert ein Zusammenwirken von Injektionsgerät 1 und Zusatzmodul 2 aus Fig.1 anhand einer schematischen Darstellung im Längsschnitt. Injektionsgerät 1 und Zusatzmodul 2 werden zur Kopplung in Richtung der Längsachse aufeinander zubewegt (Pfeil). Sobald die Führungsfläche 21b am zweiten Ende der Anpressfläche 21a auf die Auslenkkante 10c beim ersten Ende der Auflagefläche 10a trifft wird das zweite Ende des Modulelements 21 radial, in Fig.2 nach oben, ausgelenkt oder weggedrängt (Fig.2, erste Zeile). Beim weiteren Aufschieben des Zusatzmoduls auf das Injektionsgerät wird anschliessend an die Führungsfläche 21b die Anpressfläche 21a über die Auslenkkante 10c bewegt, wodurch das Modulelement weiterhin gegenüber der Längsachse verkippt oder schräggestellt ist (Fig.2, zweite Zeile). Spätestens wenn auch das erste Ende der Anpressfläche die Auslenkkante passiert hat wird das Modulelement 21 durch das maximal gespannte Federelement 23 vollflächig auf die Auflagefläche gepresst (Fig.2, dritte Zeile). Das Zusatzmodul kann auch in diesem Zustand noch weiter in Aufsetzrichtung verschoben werden, allerdings unter maximaler Gleitreibung.

Zur Unterstützung der Haftreibung in Richtung der Längsachse kann ein Sperrnocken 21c oder ein Schnapper am Zusatzmodul vorgesehen sein. Dieser verhindert ein unbeabsichtigtes Lösen des Zusatzmoduls entgegen der Aufsetzrichtung, wie dies unter Umständen durch Axialkräfte droht, welche beim Abziehen einer Nadelschutzkappe vom Injektionsgerät oder beim Aufbringen einer Nadeleinheit auf das Injektionsgerät erzeugt werden. Der Sperrnocken rastet im aufgesetzten Zustand des Zusatzmoduls in einer Ausnehmung oder Öffnung in der Gehäuseoberfläche ein und muss normalerweise durch den Anwender mittels eines Löseelements aus dieser Sperrposition gelöst werden. Der Sperrnocken kann an jeder das Injektionsgerät berührenden oder kontaktierenden Stelle des Modulgehäuses oder Modulträgers vorgesehen sein. Bevorzugt und wie in Fig.2, vierte Zeile dargestellt ist der Sperrnocken 21c am Modulelement 21 angebracht und wird dadurch über das elastische Federelement 23 des Modulelements 21 in der Sperrposition gehalten. Separate elastische Elemente oder Sperrschnapper am Zusatzmodul können somit vermieden werden.

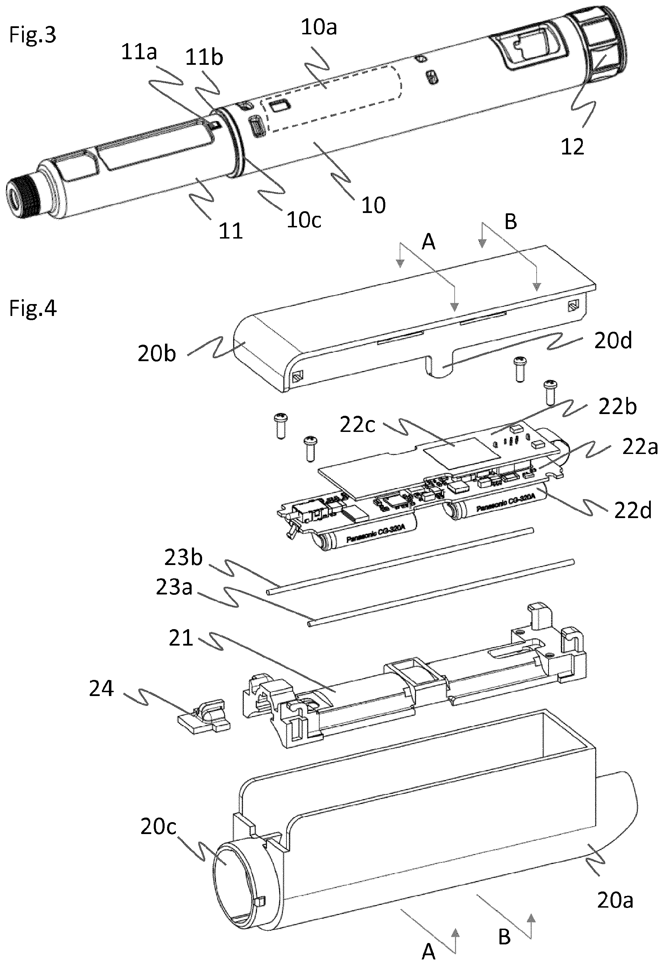

Fig.3 zeigt ein Einweg-Injektionsgerät mit variabler Dosisabgabe, umfassend ein Gerätegehäuse 10, einen Karpulenhalter 11, und einen Dosiswahlknopf 12. Der Karpulenhalter 11 weist einen Nocken 11a und eine Stoppfläche 11b auf, wobei die Stoppfläche 11b radial aussen beim nahtlosen Übergang zum Gerätegehäuse 10 von einer Auslenkkante 10c begrenzt wird. Ein distales Ende des Karpulenhalters (links in Fig.3) weist ein Gewinde auf zur Befestigung einer Injektionsnadel. Eine nicht gezeigte Gerätekappe des Injektionsgeräts 1 kann in einer den Karpulenhalter abdeckenden Position mit dem Nocken 11a eine Schnappverbindung eingehen. Eine mögliche Ausdehnung einer Auflagefläche 10a auf der Oberseite des Gerätegehäuses ist mit einer unterbrochenen Linie skizziert.

Fig.4 zeigt Einzelteile eines Zusatzmoduls nach der Erfindung in einer Explosionsdarstellung. Das Zusatzmodul umfasst ein Modulgehäuse 20 mit einem länglichen, hülsenförmigen Modulträger 20a und einem Moduldeckel 20b, welcher fest auf den Modulträger montiert, bevorzugt mit diesem verschnappt, verklebt, verschweisst, kaltverstemmt, heissverstemmt, oder verschraubt ist. Der Modulträger definiert einen Aufnahmebereich 20c zur Aufnahme des Injektionsgeräts 1 aus Fig.3 durch Einschieben des Injektionsgeräts in distale Richtung beziehungsweise durch Aufschieben des Zusatzmoduls auf das Injektionsgerät in proximaler Richtung. Das Zusatzmodul umfasst ein Modulelement 21, welches im Modulträger 20a in einer radialen Richtung senkrecht zur Längsachse beweglich aufgenommen ist. Ein erster Elektronikträger 22a ist mit dem Modulelement 21 unbeweglich verbunden, bevorzugt mit diesem verschnappt, verklebt, verschweisst, kaltverstemmt, heissverstemmt, oder verschraubt, und dient als Träger für eine Sensoreinheit. Ein zweiter Elektronikträger 22b ist am Moduldeckel montiert, mit dem ersten Träger über flexible Leiter verbunden, und dient als Träger einer Signalisierungseinheit zur Signalisierung eines Zustandes des Zusatzmoduls oder des Injektionsgerätes an einen Anwender, beispielweise mittels Leuchtdioden. Der zweite Elektronikträger 22b nimmt auch eine Eingabeeinheit auf zur Eingabe von Anwendersignalen, beispielsweise einen berührungsempfindlichen Kapazitivsensor 22c, und/oder einen Micro-USB Stecker zum Datenaustausch. Ein elastisches Federelement des Zusatzmoduls umfasst zwei gerade Federdrähte 23a, 23b, welche zwischen Moduldeckel 20b und Modulelement 21 platziert sind. Eine erste Federführung 20d als Teil des Moduldeckels 20b fixiert mittels einer an den Querschnitt des ersten Federdrahtes 23a angepassten Rille die Mitte des Federdrahts 23a gegen radiale Bewegungen. Die beiden Enden des ersten Federdrahtes 23a sind mit je einem endseitigen Schnapphaken des Modulelements verhakt. Der zweite Federdraht 23b ist ebenso in seiner Mitte und an seinen Enden mit dem Modulgehäuse verbunden beziehungsweise mit dem Modulelement verhakt. Ein axial verschiebbarer Stössel 24 vermittelt eine Anwesenheit der Gerätekappe an einen Kappendetektor auf dem ersten Elektronikträger 22a.

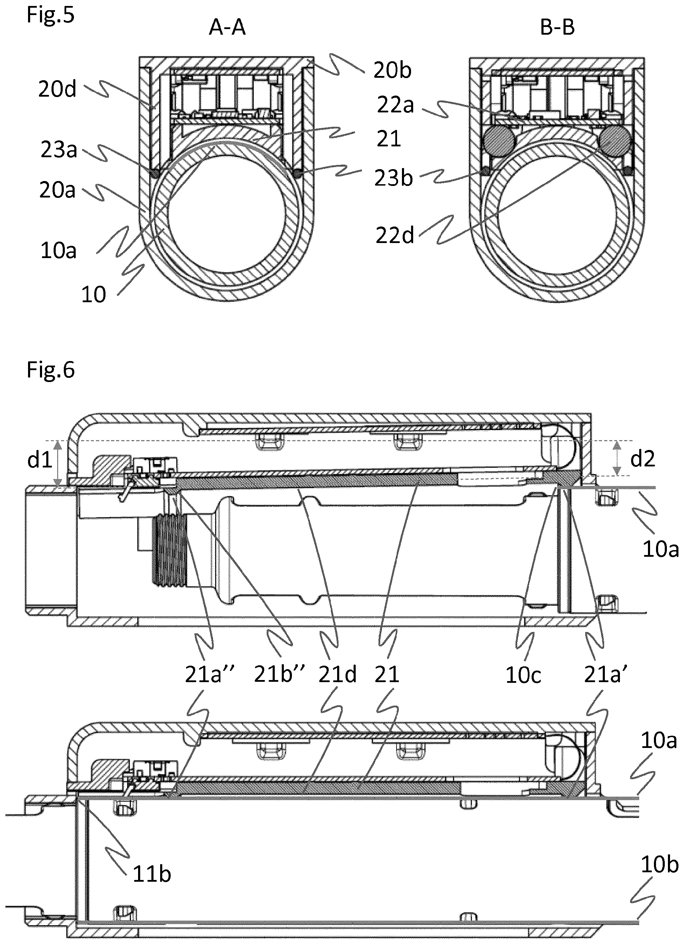

Fig.5 zeigt zwei Schnitte durch das Zusatzmodul aus Fig.4 und durch das Injektionsgerät aus Fig.3 im aufgesetzten oder gekoppelten Zustand. Der erste Schnitt erfolgt ungefähr in der Mitte des Zusatzmoduls, in der Ebene A-A gemäss Fig.4, senkrecht zur Längsachse. Zwei Federführungen 20d als Teil des Moduldeckels 20b sind erkennbar, sie bilden zusammen mit dem Modulträger 20a ein starres Verbindungselement. Durch die Enden der Federdrähte werden Federkräfte der vorgespannten Federdrähte in Fig.5 nach unten auf das Modulelement übertragen, wodurch in der Summe eine radiale Anpresskraft des Modulelements 21 auf die Auflagefläche 10a resultiert. Die Federführungen 20d können in Längsrichtung auch an einer anderen Stelle als in der Mitte des Modulelements positioniert werden, dadurch wird der Verlauf einer durch den Anwender beim Aufsetzen des Zusatzmoduls aufzuwendenden Kraft unmittelbar beeinflusst.

Der zweite Schnitt erfolgt neben der Mitte des Zusatzmoduls, in der Ebene B-B gemäss Fig.4, senkrecht zur Längsachse. Entsprechend ihrer durchgebogenen Längsform befinden sich die Querschnitte der Federdrähte 23a, b an dieser Stelle leicht höher als im ersten Schnitt. Unterhalb des ersten Elektronikträgers 22a sind platzsparend vier kleine, zylinderförmige Energiespeicher 22d angeordnet, bespielweise Lithium Polymer Batterien von 20 mm Länge, 3.6 mm Breite, und 3.8 V Spannung. Aussparungen oder Einschnitte im Modulelement 21 können die Anordnung der Energiespeicher an dieser Stelle weiter begünstigen.

Fig.6 zeigt einen Längsschnitt durch eine Symmetrieebene des Zusatzmoduls aus Fig.4, sowohl zu Beginn der Aufsetzbewegung (oben) als auch am Ende der Aufsetzbewegung, in finalem Zustand (unten). Die Anpressfläche 21a umfasst hier zwei Abschnitte 21a', 21a" an den beiden Enden des Modulelements 21, welche in Längsrichtung durch eine gegenüber der Anpressfläche leicht zurückgesetzte oder abgesenkte Verbindungsfläche 21d getrennt sind. In der ersten Position hat die erste Anpressfläche 21a' die Auslenkkante 10c am Ende der Auflagefläche 10a bereits überwunden, und rutscht selbst auf der Auflagefläche 10a zumindest so lange bis die Verbindungsfläche 21d auf die Auslenkkante 10c trifft. Die dadurch resultierende Verkippung des Modulelements 21 wird durch die zwei unterschiedlich langen Doppelpfeile zwischen den beiden gestrichelten Hilfslinien illustriert, welche parallel sind zur Längsachse (obere Hilfslinie) und zur Anpressfläche (untere Hilfslinie). Im finalen aufgesetzten Zustand (Fig.6 unten) hat auch die zweite Anpressfläche 21a" dank ihrer Führungsfläche 21b" die Auslenkkante 10c überquert du steht jetzt ebenfalls auf der Auflagefläche 10a auf. Demgegenüber kontaktiert die Verbindungsfläche 21d die Auflagefläche 10a im aufgesetzten Zustand nicht. In dieser Position ist auch der Anschlag einer nach proximal gerichteten Anschlagsfläche des Zusatzmoduls an der nach distal gerichteten Stoppfläche 11b des Karpulenhalters des Injektionsgeräts erkennbar.

**BEZUGSZEICHENLISTE**

| | | | |
|---|---|---|---|
| 1 | Injektionsgerät | 20c | Aufnahmebereich |
| 10 | Gerätegehäuse | 20d | Federführung |
| 10a | Auflagefläche | 21 | Modulelement |
| 10b | Gegenfläche | 21a | Anpressfläche |
| 10c | Auslenkkante | 21b | Führungsfläche |
| 11 | Karpulenhalter | 21c | Sperrnocken |
| 11a | Nocken | 22a, 22b | Elektronikträger |
| 11b | Stoppfläche | 22c | Berührungssensor |
| 12 | Dosiswahlknopf | 22d | Batterie |
| 2 | Zusatzmodul | 23 | Federelement |
| 20 | Modulgehäuse | 23a, b | Federdrähte |
| 20a | Modulträger | 24 | Stössel |
| 20b | Moduldeckel | | |

## Patentansprüche

1. Zusatzmodul (2) zum lösbaren Aufsetzen auf ein Injektionsgerät (1) mit einer Längsachse und einem Gerätegehäuse (10), wobei das Gerätegehäuse des Injektionsgeräts eine erste Seite mit einer zur Längsachse parallelen Auflagefläche (10a) und eine der ersten Seite gegenüberliegende zweite Seite aufweist, und wobei das Zusatzmodul umfasst:
- ein in einem Modulgehäuse (20) senkrecht zur Längsachse verschiebbar gelagertes Modulelement (21) mit einer an die Auflagefläche (10a) angepassten Anpressfläche (21a), und
- einen in einem aufgesetzten Zustand des Zusatzmoduls an der zweiten Seite des Gerätegehäuse abstützenden Federmechanismus (20a, 20b, 20d; 23) zum Anpressen der Anpressfläche (21a) auf die Auflagefläche (10a).

2. Zusatzmodul nach Anspruch 1, umfassend eine Sensoreinheit zum Detektieren von Bewegungen des Injektionsgerätes, wobei die Sensoreinheit unbeweglich auf dem Modulelement (21) befestigt ist.

3. Zusatzmodul nach Anspruch 1 oder 2, wobei der Federmechanismus so ausgebildet ist, dass das Modulelement (21) bei einer Aufsetzbewegung des Zusatzmoduls auf das Injektionsgerät um eine Achse senkrecht zur Längsrichtung verkippt und dass erst gegen Ende der Aufsetzbewegung die Anpressfläche (21a) vollflächig auf der Auflagefläche (10a) aufliegt.

4. Zusatzmodul nach Anspruch 3, **dadurch gekennzeichnet dass** ein erstes Ende der Anpressfläche (21a) des Modulelements (21) und/oder eine Auslenkkante (10c) an einem zweiten Ende der Auflagefläche (10a) des Gerätegehäuses eine Führungsfläche (21b) aufweisen, welche die Verkippung der Anpressfläche bewirkt.

5. Zusatzmodul nach Anspruch 1 oder 2, umfassend einen Aufnahmebereich (20c) zur Aufnahme des Injektionsgerätes, wobei der Federmechanismus mit einem starren Verbindungselement (20a, 20b, 20d) an der zweiten Seite abstützt und mit einem elastischen Federelement (23) zwischen dem starren Verbindungselement und dem verschiebbar gelagerten Modulelement (21) eine Anpresskraft erzeugt.

6. Zusatzmodul nach Anspruch 5, wobei das elastische Federelement (23) an mehreren in Richtung der Längsachse verteilten Stellen das Verbindungselement und das Modulelement kontaktiert.

7. Zusatzmodul nach Anspruch 1 oder 2, zum Aufsetzen auf ein rotationssymmetrisches Injektionsgerät, umfassend ein Rotationsbegrenzungselement zur rotativen Positionierung von Zusatzmodul und Injektionsgerät.

8. Zusatzmodul nach Anspruch 1 oder 2, wobei das Modulelement (21) bezüglich einer die Längsachse umfassenden Symmetrieebene zumindest näherungsweise symmetrisch ausgebildet ist, die Anpressfläche (21a) einen konkaven Querschnitt aufweist, und das Zusatzmodul einen ebenen Elektronikträger (22a) umfasst, **dadurch gekennzeichnet, dass** der Federmechanismus aufweist zwei zur Symmetrieebene symmetrisch und zwischen Anpressfläche und Elektronikhalter angeordnete Federelemente (23a, b).

9. Zusatzmodul nach Anspruch 1 oder 2, umfassend einen Gerätekappensensor (24) zur Detektion einer Entfernung einer Gerätekappe des Injektionsgerätes, und einen Berührungssensor (22c) zur Detektion einer Berührung des Zusatzmoduls durch einen Anwender.

10. Zusatzmodul nach Anspruch 1 oder 2, umfassend eine Anschlagfläche zur Begrenzung einer axialen Relativbewegung von Zusatzmodul und Injektionsgerät in eine Aufsetzrichtung.

11. Zusatzmodul nach Anspruch 10, wobei die Relativbewegung eine Aufsetzbewegung des Zusatzmoduls auf das Injektionsgerät in proximaler Richtung ist und wobei die Anschlagfläche an einem Flansch (11b) des Injektionsgerätes anschlägt.

12. Injektionssystem mit einem Injektionsgerät (1) und einem auf das Injektionsgerät aufgesetzten Zusatzmodul (2) nach Anspruch 2, wobei ausschliesslich die durch den Federmechanismus auf die Auflagefläche (10a) angepresste Anpressfläche (21a) eine axiale Fixierung von Zusatzmodul und Injektionsgerät bewirkt.
